# FASCICULE DE BREVET EUROPEEN

(11) **EP 0 468 331 B1**
(45) Date de publication et mention de la délivrance du brevet: **05.10.1994**
(21) Numéro de dépôt: 91111747.1
(22) Date de dépôt: 15.07.1991
(51) Int. Cl.: H01H 35/24, A61M 5/168

(54) **Dispositif de détection d'une surpression à l'intérieur d'un tuyau et pompe munie d'un tel dispositif**
Überdruckdetektor und damit versehene Pumpe
Overpressure detector and pump incorporating the same

(30) Priorité: 23.07.1990 FR 9009477
(43) Date de publication de la demande: 29.01.1992
(73) Titulaire: ASULAB S.A., CH-2501 Bienne (CH)
(72) Inventeur: Gagnebin, Eric, CH-2074 Marin (CH)
(74) Mandataire: Barbeaux, Bernard

(56) Documents cités:
- WO-A-86/07266
- DE-A- 3 035 703

## Description

La présente invention concerne un dispositif de détection d'une surpression à l'intérieur d'un tuyau et une pompe munie d'un tel dispositif. Bien que l'application de ce dispositif de détection ne soit pas limité à un type de tuyau particulier, il s'adapte plus particulièrement à ceux de faible diamètre.

Dans les systèmes d'alimentation en fluide comprenant une pompe faisant progresser un liquide ou un gaz à l'intérieur d'un tuyau, il peut arriver que ce tuyau se bouche partiellement ou totalement, ce qui a pour effet d'entraîner une surpression à l'intérieur de celui-ci. Dans de nombreux cas, il est impératif de détecter cette surpression et d'arrêter la pompe le plus rapidement possible, afin d'éviter, d'une part, que le tuyau ne se déforme ou même n'éclate et d'autre part, que la pompe continue de fonctionner inutilement.

On comprendra que ce type de problème est d'autant plus grave lorsqu'il s'agit d'une pompe médicale, par exemple une pompe péristaltique, telle que celle décrite dans la demande de brevet FR-9 003 869 appartenant à la Demanderesse. Cette pompe médicale est destinée à faire progresser un médicament sous forme liquide dans un tuyau reliant un réservoir à une aiguille hypodermique d'injection. Si ce tuyau se bouche, le patient risque de ne plus recevoir son médicament, ce qui peut perturber le traitement, être inconfortable pour lui, voire même, dangereux. De plus, la pompe risque d'être endommagée.

Il est donc nécessaire de réaliser un détecteur déclenchant le fonctionnement d'une alarme ou même interrompant le fonctionnement de la pompe dès que la surpression dans le tuyau dépasse un certain seuil.

En outre, dans la mesure où ces pompes médicales sont souvent des pompes à usage unique, jetables au bout d'un certain nombre de jours d'utilisation, on cherchera à réaliser un détecteur également à usage unique. La réalisation d'un détecteur à usage unique nécessite bien évidement que celui-ci soit d'un très faible coût de revient.

Enfin, il faut que ce détecteur soit de faibles dimensions pour pouvoir, par exemple, être intégré directement dans la pompe.

On connaît déjà des dispositifs comprenant un piston ou un clapet placé entre un fluide et un moyen élastique de retenue, ce piston ou ce clapet étant relié à un moyen de détection de son déplacement. On connaît en outre le principe du tube de Bourdon utilisé dans les manomètres classiques. Enfin, on connaît aussi des systèmes de détection de pression utilisant des capteurs a quartz ou des jauges de contrainte. Tous ces dispositifs sont malheureusement volumineux, coûteux et ne sont pas forcément adaptés à un usage médical.

On connaît également du document WO-A-86/07266 un dispositif de détection d'une surpression. Ce dispositif comprend une membrane présentant une première position stable correspondant à une pression normale et une seconde position stable correspondant à une surpression. Pour détecter si la membrane est dans la première position stable ou dans la seconde position stable, ce document prévoit un système optique comprenant une source de lumière et un détecteur de lumière permettant de détecter si la membrane est située dans le chemin optique entre la source de lumière et le détecteur de lumière. La construction de ce dispositif est peu aisée et son coût de fabrication relativement élevé.

L'invention a donc pour but de remédier à ces inconvénients.

A cet effet, l'invention a pour objet un dispositif de détection d'une surpression dans un tuyau dans lequel circule un fluide, ce dispositif étant destiné à générer un signal dès le franchissement d'un seuil de surpression.

Conformément à l'invention, ce dispositif comprend :
- des moyens de dérivation d'une partie dudit fluide circulant provenant du tuyau,
- au moins un tube dans lequel est placé au moins un organe d'obturation, ce tube étant relié à son extrémité amont auxdits moyens de dérivation, l'un des éléments formant ledit tube et ledit organe d'obturation étant élastiquement déformable pour retenir ledit organe d'obturation dans le tube, dans une position de fonctionnement normal, grâce aux forces de frottement engendrées à leurs surfaces de contact, l'organe d'obturation quittant la position de fonctionnement normal lorsque survient dans le tuyau une surpression capable de vaincre lesdites forces de frottement, et
- des moyens de détection du déplacement dudit organe d'obturation générant ledit signal dès qu'ils détectent le déplacement de l'organe d'obturation.

Grâce à ces caractéristiques de l'invention, on détecte très facilement la surpression ou le dépassement d'un seuil de surpression dans le tuyau, puisque cette surpression se répercute immédiatement dans le tube du détecteur. En effet, lors du fonctionnement normal la pression du fluide se trouvant dans le tube en amont de l'organe obturateur est équilibrée par la pression de l'air se trouvant en aval de cet organe obturateur et par les forces de frottement exercées par l'organe obturateur sur le tube. Au contraire, en cas de surpression, la pression exercée par le fluide est supérieure à celle de l'air et aux forces de frottement. Au delà d'une certaine surpression, l'organe obturateur se déplace à l 'intérieur du tube, vers l 'aval, en direction des moyens de détection du déplacement.

Ce dispositif de détection comprend un faible nombre de pièces et est donc peu coûteux à réaliser.

L'invention sera mieux comprise à la lecture de la description suivante donnée uniquement à titre d'exemple et faite en référence aux dessins joints dans lesquels :
- les figures 1A et 2A représentent des vues en coupe d'un premier et d'un second mode de réalisation du dispositif de détection selon l'invention,
- les figures 1B et 2B représentent des vues en coupe partielles du dispositif de détection respectivement selon les figures 1A et 2A, mais avant le fonctionnement de ce dispositif, à savoir en position de repos,
- la figure 3 représente une vue en coupe selon la ligne III-III de la figure 2A,
- les figures 4, 5 et 6 représentent respectivement des vues en coupe des troisième, quatrième et cinquième modes de réalisation de l'invention,
- la figure 7 représente une vue en coupe d'une pompe médicale comprenant un dispositif de détection selon l'invention.

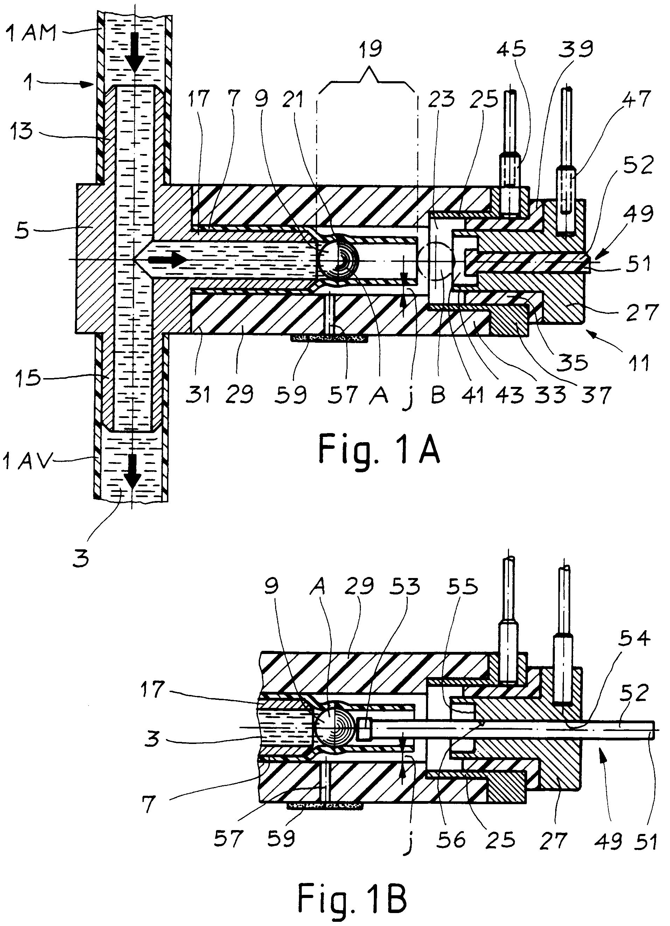

Un premier mode de réalisation du dispositif selon l'invention est illustré en figure 1A. Ce dispositif est destiné d'une part à détecter une surpression ou le franchissement d'un seuil de surpression dans un tuyau 1 dans lequel circule un fluide 3, et d'autre part à générer un signal dès que ce seuil est franchi. Le signal généré est le plus souvent un signal électrique.

Dans ce mode de réalisation, le fluide 3 est nécessairement un liquide conducteur.

Le dispositif de détection comprend des moyens de dérivation 5 d'une partie du liquide 3 se trouvant dans le tuyau 1, un tube 7 dans lequel est placé un organe obturateur 9 ajusté à frottement et des moyens de détection 11 du déplacement de cet organe 9.

Les moyens de dérivation 5 sont constitués par un raccord en T, réalisé en matière plastique, par exemple, pour des raisons de faible coût. Ce raccord 5 présente une branche d'entrée 13 et une branche de sortie 15 toutes deux coaxiales, ainsi qu'une troisième branche de dérivation 17 perpendiculaire aux deux autres. Dans la description qui suit, les termes amont et aval sont définis par rapport au sens de circulation du fluide 3. Le tuyau 1 réalisé par exemple en silicone est coupé en deux parties. La partie amont 1 AM, reliée à la pompe (non représentée) qui fait circuler le liquide 3, est engagée sur la branche d'entrée 13. La partie aval 1 AV menant vers la sortie du dispositif est engagée sur la branche de sortie 15. Par ailleurs, la partie amont du tube 7 est engagée sur la branche de dérivation 17.

De préférence, ce tube 7 est réalisé en matière élastique déformable, par exemple en silicone. Il est d'une longueur suffisante pour pouvoir recouvrir la totalité de la branche 17 et donc être fixé solidement sur celle-ci, tout en pouvant également laisser une portion libre 19, en aval, dans laquelle l'organe d'obturation 9 peut se déplacer.

Cette organe d'obturation 9 est de préférence une bille comme représenté en figure 1, mais pourrait également avoir la forme d'un corps de révolution tel qu'un cylindre, par exemple. La bille 9 présente un diamètre légèrement supérieur au diamètre interne du tube 7 de façon à assurer au niveau de la surface de contact 21 une étanchéité suffisante pour empêcher le passage du liquide 3. En effet, la portion 19 du tube 7 se déforme et épouse les contours de la bille 9 qui elle est indéformable. On empêche ainsi que le liquide 3 se trouvant en amont de la bille 9 ne pénètre de façon incontrôlée dans la partie aval 19 du tube et vienne activer et déclencher de façon inopinée les moyens de détection.

Les moyens 11 de détection du déplacement de la bille 9 comprennent une chambre 23 dans laquelle sont disposées deux électrodes 25 et 27. L'extrémité aval 19 du tube 7 débouche dans cette chambre. Ladite chambre 23 est délimitée par une douille 29 de préférence cylindrique, dont l'extrémité amont 31 est serrée sur la branche de dérivation 17 et sur la partie amont du tube 7 qui est prise en sandwich entre la douille 29 et la branche 17. L'extrémité aval 33 de la douille 29 est fermée par les deux électrodes 25 et 27. Le diamètre interne de cette douille 29 est supérieur au diamètre externe de la portion 19 du tube 7, de façon a laisser un espace annulaire j entre ces deux éléments et à permettre au tube 7 de se déformer radialement lors du passage de la bille 9 dans celui-ci. En outre, la longueur de la douille 29 est supérieure à celle du tube 7 pour que la bille puisse sortir complètement de ce dernier. La douille 29 est réalisée de préférence en matière plastique transparente, par exemple en plexiglas ou en polycarbonate, afin de permettre la visualisation de la bille 9.

La première électrode 25 à la forme d'un manchon, de préférence cylindrique, définissant la partie aval des parois de la chambre 23. On pourrait également envisager que cette électrode s'étende sur la totalité des parois de la chambre 23. La seconde électrode 27 est coaxiale à la première et logée à l'intérieur de celle-ci. Cette seconde électrode 27 est séparée de la première par une bague isolante 35 réalisée, par exemple, en "Delrin" ou en "Nylon" (marques déposées).

La première électrode 25 présente un épaulement annulaire 37 et est chassée dans la douille 29 jusqu'à ce que cet épaulememt bute contre l'extrémité de ladite douille. La bague isolante 35 présente également un épaulement annulaire 39 et est montée de la même façon dans la première électrode 25. Enfin, la seconde électrode 27 est chassée dans la bague isolante 35. Cette seconde électrode 27 présente une cavité 41 débouchant dans la chambre 23, cette cavité étant définie par une paroi annulaire 43. Cette seconde électrode 27 est suffisamment éloignée de l'extrémité du tube 7 pour que la bille 9 puisse sortir dans la chambre 23.

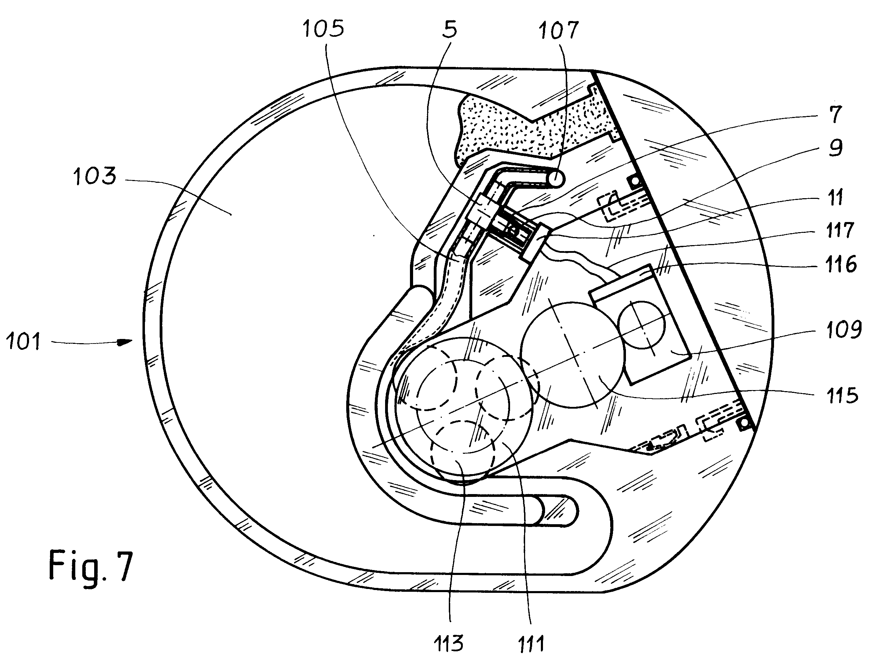

Les deux électrodes sont réalisées en métal conducteur par exemple, en maillechort ou en laiton nickelé. Ces deux électrodes 25 et 27 sont reliées chacune à une borne de connexion respectivement 45 et 47. Ces bornes 45, 47 sont reliées à un circuit électrique (représenté uniquement en figure 7) capable de générer un signal électrique à la suite de la détection du déplacement de la bille 9. Ce signal électrique peut commander soit une alarme, soit l'arrêt immédiat et irréversible du moteur de la pompe qui, en fonctionnement normal fait circuler le fluide 3 dans le tuyau 1.

Dans le détecteur selon l'invention, l'organe d'obturation 9 (ici la bille) se déplace entre une position initiale A occupée lors du fonctionnement normal et une position B occupée dès le franchissement d'un certain seuil de surpression dans le tuyau 1. Ce seuil de surpression est défini en fonction du diamètre de la bille 9 et du diamètre interne du tube 7. Plus le diamètre de la bille 9 sera élevé par rapport au diamètre interne du tube 7 et plus le seuil de surpression à atteindre pour que la bille 9 se déplace, sera élevé. Ce seuil est également fonction de l'élasticité du tube 7. Dans la position initiale A, la bille 9 se trouve dans le tube 7, sensiblement à la sortie de la branche de dérivation 17. Dans la position finale B, elle est hors du tube 7 et se trouve dans la chambre 23. Ce dispositif de détection est donc à usage unique puisqu'il n'est pas possible de réintroduire la bille 9 dans le tube 7.

Toutefois, lors de la mise en marche de l'ensemble du dispositif et notamment lors du premier remplissage du tuyau 1 par le liquide 3, il peut apparaître dans ce tuyau 1 une surpression supérieure au seuil qui est fixé pour déterminer une anomalie de fonctionnement devant être signalée au cours du fonctionnement. Il est évident que dans ce cas, cette surpression au remplissage ne doit pas provoquer l'éjection de la bille 9 du tube 7. En conséquence, dans un autre mode de réalisation visible à la figure 1B, on peut prévoir des moyens de maintien 49 de la bille 9 dans la position initiale A pendant le remplissage initial du tuyau 1.

Ces moyens de maintien 49 comprennent un piston 51 muni d'une tige 52 et d'une tête 53. La tige 52 est disposée coaxialement à la seconde électrode 27 et peut coulisser librement à l'intérieur d'un trou 54 prévu à cet effet dans ladite électrode 27. Ce trou 54 débouche dans la cavité 41 et définit à cet endroit un épaulement 55. En outre, la tige 52 présente une zone de moindre résistance 56 qui permet de la briser après son retrait et les opérations de remplissage. Le résultat apparaît sur la figure 1A. La tête 53 présente un diamètre supérieur au diamètre de la tige 52 ce qui empêche le retrait total de la partie avant du piston.

Enfin, on prévoit dans la douille 29, un évent 57 permettant l'évacuation de l'air se trouvant dans la chambre 23 et la cavité 41, lorsque la bille 9 est expulsée du tube 7. L'orifice de sortie de cet évent 57 est de préférence muni d'une pastille buvard 59 permettant d'éviter que le liquide 3 ne sorte du dispositif détection, lorsque la bille 9 est en position B.

Le fonctionnement du dispositif de détection va maintenant être expliqué de façon détaillée.

La figure 1B illustre la position initiale du piston 51 lorsque l'on remplit le tuyau 1 pour la première fois. Ce piston 51 est disposé de façon que sa tête 53 soit au contact de la bille 9, et maintienne celle-ci dans sa position initiale A en évitant tout déplacement intempestif. Lorsque l'on souhaite utiliser le détecteur, on tire le piston 51 vers l'extérieur jusqu'à ce que la tête 53 soit au fond de la cavité 41 et bute contre l'épaulement 55 et l'on rompt l'extrémité sortante du piston 51 au niveau de la zone de moindre résistance 56 prévue à cet effet. Le dispositif de détection est alors prêt à fonctionner (figure 1A).

Lorsqu'une surpression apparaît dans le tuyau 1, ce qui est le cas si ledit tuyau se bouche dans sa partie aval 1 AV, cette surpression se répercute dans la branche de dérivation 17. Lorsque cette surpression dépasse un seuil prédéterminé, la bille 9 est chassée hors du tube 7 vers la chambre 23. Le liquide 3 qui est conducteur se répand alors dans ladite chambre et relie entre elles les deux électrodes 25 et 27, ce qui a pour effet de fermer le circuit électrique auquel elles sont reliées. On déclenche aussi le signal électrique commandant, par exemple, une alarme. On notera que la forme particulière des électrodes 25 et 27 et de la cavité 41 permettent d'avoir une surface de contact électrique relativement importante par rapport aux dimensions desdites électrodes.

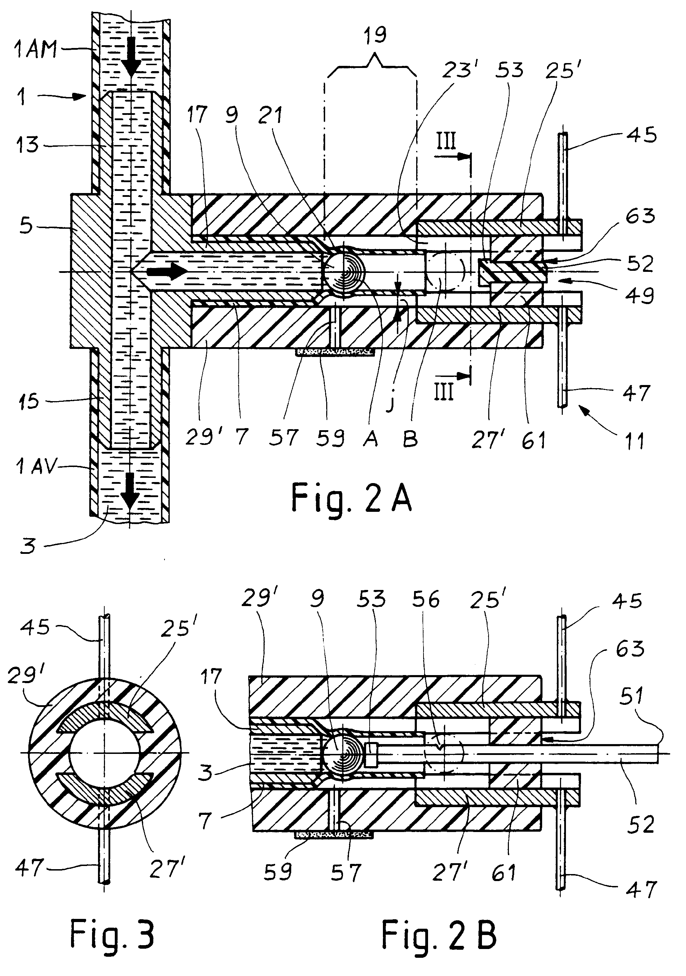

La figure 2A illustre un détecteur similaire à celui représenté à la figure 1A, dans lequel seules les électrodes ont une forme différente.

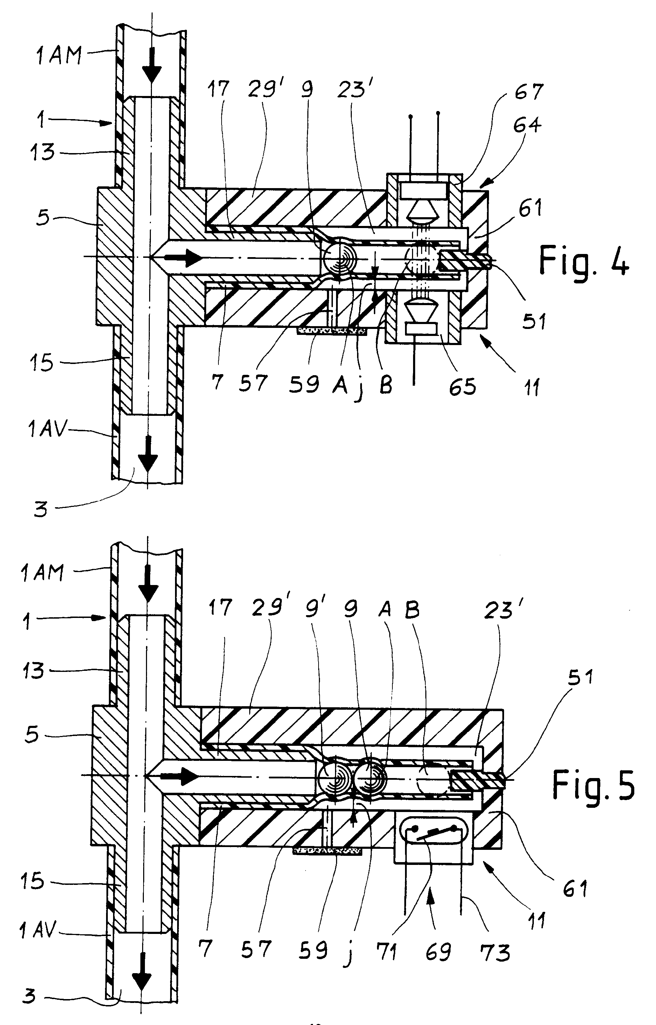

Les éléments identiques des deux détecteurs portent les mêmes références. Les deux électrodes sont référencées 25′ et 27′, elles sont symétriques, ont la forme de portions de cylindre coupées dans le sens de la longueur (voir figure 3) et épousent la forme de la douille cylindrique, référencée ici 29′. Ces deux électrodes 25′ et 27′ et la douille 29′ définissent une chambre 23′. Dans ce mode de réalisation, les deux électrodes 25′ et 27′ sont surmoulées dans la douille 29′ en matière plastique sous forme d'inserts. En conséquence, l'extrémité aval de cette douille 29′ est fermée par une paroi 61 constituée par un bouchon électriquement isolant définissant le fond de la chambre 23. Le centre de cette paroi 61 est percé d'une ouverture 63 permettant le passage du piston 51. Enfin, les deux extrémités aval des électrodes 25′ et 27′ dépassent hors de la douille 29′ pour permettre la fixation des bornes de connexion respectivement 45 et 47.

La figure 2B similaire à la figure 1B illustre la position du piston 51 lorsque celui-ci maintient la bille 9, lors du remplissage.

Dans un dispositif de détection réalisé par la Demanderesse, les dimensions et valeurs suivantes ont été utilisées, ces données n'étant présentées ici qu'à titre d'exemple purement illustratif.

Le tube 7 réalisé par exemple en polymère silicone présente un diamètre interne de 1,47 mm et un diamètre externe de 1,96 mm. La surpression nécessaire pour éjecter la bille 9 hors du tube 7, est de 0,4 à 0,5 bar (0,4 à 0,5.10⁵ Pa) lorsque le diamètre de la bille est de 1,59 mm et de 0,6 à 0,7 bar (0,6 à 0,7. 10⁵ Pa) lorsque ce diamètre est de 1,65 mm.

Dans le mode de réalisation représenté sur les figures 1A, 1B, la première électrode (l'électrode externe 25) présente une surface active de contact électrique de 16,50 mm² et l'électrode interne 27 une surface active de 15,12 mm².

Dans le mode de réalisation des figures 2A et 2B, la surface active de contact de chaque électrode 25′ et 27′ est de 7,63 mm².

Le troisième mode de réalisation de l'invention, illustré en figure 4, va maintenant être décrit. Dans ce dispositif de détection, seuls les moyens de détection 11 du déplacement de la bille 9 sont différents. Les autres éléments sont identiques à ceux des figures 2A et 2B et portent donc les mêmes références.

Dans ce mode de réalisation, le fluide 3 peut être un liquide ou un gaz non conducteur. De plus, il n'est pas nécessaire que l'organe d'obturation, dans ce cas la bille 9, présente obligatoirement au niveau de sa surface de contact 21, un diamètre supérieur au diamètre interne du tube 7 pour assurer l'étanchéité parfaite. Ces deux diamètres peuvent être identiques. On s'assure ainsi simplement que la bille 9 est maintenue par frottement dans sa position initiale A. On notera que le tube 7 se prolonge sensiblement jusqu'à la paroi arrière 61 de la douille 29′.

Les moyens de détection 11 du déplacement de la bille 9 comprennent un capteur photoélectrique 64 muni d'une source lumineuse 65 et d'un récepteur 67. Ce capteur 64 est disposé sur le trajet de la bille 9. Plus précisément, l'émetteur 65 et le récepteur 67 sont disposés dans les parois de la douille 29′, de part et d'autre du tube 7 et en aval de la bille 9.

Sous l'action d'une surpression exercée par le fluide 3, la bille 9 se déplace de sa position initiale A à sa position finale B, où elle s'interpose entre la source 65 et le récepteur 67, en activant ainsi le capteur 64. Le récepteur peut alors émettre un signal électrique de détection du passage de la bille 9.

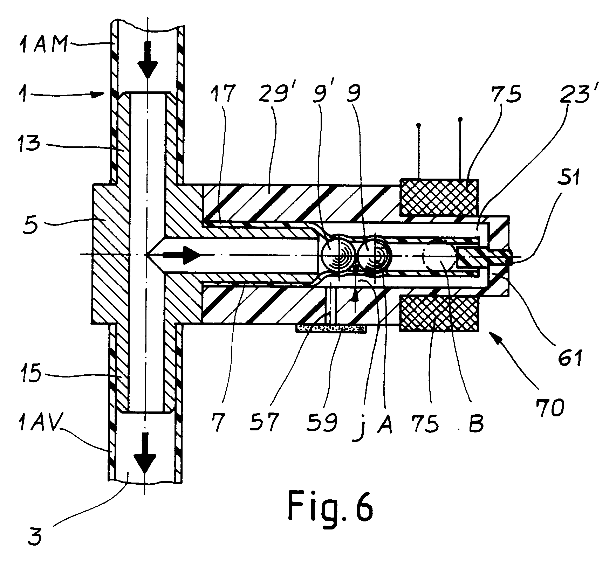

Les figures 5 et 6 illustrent deux autres variantes de réalisation, dans lesquelles le capteur photoélectrique 64 est remplacé respectivement par un capteur magnétique 69 et par un capteur électromagnétique 70. Dans ces deux cas, le fluide 3 est indifféremment un liquide ou un gaz.

Sur la figure 5, le capteur magnétique 69 disposé dans la douille 29′, est un contact comprenant une lame métallique aimantée 71 mobile angulairement. La bille 9 est métallique. Lors du passage de la bille 9 devant cette lame 71, cette dernière bascule et ferme le circuit électrique 73 déclenchant ainsi un signal électrique.

On pourrait également prévoir que la lame 71 soit métallique mais non aimantée et que la bille 9, (ou de préférence un cylindre) soit aimantée.

Sur la figure 6, le capteur électromagnétique 70 est constitué par une bobine 75 disposée autour du tube 7 et autour de la douille 29′.

Sur ces figures 5 et 6, on a représenté deux billes 9 dont l'utilité sera expliquée lors de la description de la figure 7.

La figure 7 illustre un exemple d'application particulière du dispositif de détection selon l'invention. Cette figure illustre un dispositif médical, ici une pompe péristaltique 101 destinée à injecter à un patient un médicament qui constitue ici le fluide circulant 3.

Ce dispositif 101 comprend un réservoir 103 de médicament, relié par un tuyau d'alimentation 105 à un orifice de sortie 107 où est fixée une aiguille hypodermique (non représentée). Un moteur 109 actionne une pompe péristaltique 111 munie de galets presseurs 113, par l'intermédiaire d'une roue dentée 115.

On dispose le dispositif de détection selon l'invention entre la pompe 111 et l'orifice de sortie 107. On retrouve sur la figure 7, les moyens de dérivation 5, le tube 7, la bille 9 et les moyens de détection 11 du passage de ladite bille. Ces derniers sont reliés au moteur 109 ainsi qu'a une alarme 116, par un circuit électrique 117. Si une surpression apparaît dans le tuyau 105, le signal électrique généré par le circuit électrique 117 commande l'alarme 116 et l'arrêt immédiat du fonctionnement de la pompe 111.

Dans le cas d'applications médicales comme pour cette pompe, on réalisera le dispositif de détection en des matériaux compatibles avec un usage médical. Ainsi, la bille 9 sera réalisée en matériau médical, stérilisable et neutre chimiquement vis-à-vis du fluide 3, par exemple, en matériau plastique tel que le PTFE (polytétrafluoréthylène), en verre, en céramique ou même éventuellement en un métal tel que l'or ou le platine.

Lorsque l'on utilise les dispositifs des figures 5 et 6 dans ce type d'application médicale, on placera deux billes 9 dans le tube 7. La première bille amont 9′ sera réalisée en matière plastique médicale et la bille aval 9 sera bien évidemment métallique ou aimantée. Ainsi, le médicament ne sera en contact qu'avec la bille amont 9′ réalisée en matériau stérilisable et lorsque les deux billes 9, 9′ seront chassées par la surpression, c'est la bille métallique 9 qui déclenche les capteurs magnétiques ou électromagnétiques.

L'exemple chiffré qui a été donné précédemment s'applique plus particulièrement à cette pompe médicale de faible dimension. Toutefois, on pourrait augmenter les dimensions du dispositif de détection dans d'autres applications.

Dans tous les modes de réalisation qui ont été décrits précédemment, l'organe d'obturation 9, 9′ est réalisé en un matériau indéformable tandis que le tube 7 est lui réalisé en matière élastique déformable afin de se déformer légèrement autour de l'organe 9, 9′ et d'assurer ainsi, non seulement le blocage de cet organe dans sa position initiale A mais également, l 'étanchéité. Toutefois, on pourrait arriver au même résultat en réalisant le tube 7 en un matériau indéformable et l'organe d'obturation 9, 9′ en un matériau expensible, élastique qui se déformerait de façon à occuper l'intérieur du tube 7.

Enfin, on pourrait concevoir de placer sur un même tuyau 1, plusieurs dispositifs de détection en parallèle, présentant des rapports différents entre le diamètre de la bille 9 et le diamètre interne du tube 7 ou présentant des tubes 7 d'élasticité différente. On obtiendrait ainsi des dispositifs se déclenchant à des seuils de surpression différents. On pourrait ainsi prévoir qu'un premier dispositif de détection déclenche une alarme lorsqu'une faible surpression apparaît et qu'un deuxième arrête le moteur de la pompe en cas de forte surpression.

## Revendications

1. Dispositif de détection d'une surpression dans un tuyau (1) dans lequel est susceptible de circuler un fluide (3), ce dispositif étant destiné à générer un signal dès le franchissement d'un seuil de surpression et comprenant :
- des moyens de dérivation (5) d'une partie dudit fluide (3) se trouvant dans le tuyau (1),
- au moins un tube (7) dans lequel est placé au moins un organe d'obturation (9; 9′), ce tube étant relié à son extrémité amont audits moyens de dérivation (5), l'un des éléments formant ledit tube (7) et ledit organe d'obturation (9; 9′) étant élastiquement déformable pour retenir ledit organe d'obturation dans le tube, dans une position (A) de fonctionnement normal, grâce aux forces de frottement engendrées à leurs surfaces de contact (21), l'organe d'obturation (9; 9′) quittant la position (A) de fonctionnement normal lorsque survient dans ledit tuyau (1) une surpression capable de vaincre lesdites forces de frottement, et
- des moyens de détection (11) du déplacement dudit organe d'obturation (9; 9′) générant ledit signal dès qu'ils détectent le déplacement de l'organe d'obturation (9, 9′).

2. Dispositif de détection selon la revendication 1, caractérisé en ce que ledit tube (7) est réalisé en une matière élastique et en ce que ledit organe d'obturation (9, 9′) présente un corps de révolution indéformable ajusté à frottement dans ledit tube (7).

3. Dispositif de détection selon la revendication 2, caractérisé en ce que ledit corps de révolution (9, 9′) présente à la surface de contact (21) avec ledit tube (7) un diamètre au moins égal au diamètre de ce tube.

4. Dispositif de détection selon la revendication 2, caractérisé en ce que le diamètre du corps de révolution (9, 9′), au niveau de la surface de contact (21) avec ledit tube (7), est supérieur au diamètre interne de ce dernier, pour empêcher le fluide (3) se trouvant dans la partie amont du tube (7) de pénétrer dans la partie aval (19) de ce dernier.

5. Dispositif de détection selon l'une quelconque des revendications 1 à 4, caractérisé en ce que l'organe d'obturation (9, 9′) a la forme d'une bille.

6. Dispositif de détection selon l'une quelconque des revendications 1 à 5, caractérisé en ce que les moyens de dérivation (5) comprennent un raccord en T dont deux branches (13; 15) sont adaptées pour être connectées dans ledit tuyau (1; 1AM, 1AV) et dont la troisième branche (17) est connectée à l'extrémité amont du tube (7).

7. Dispositif de détection, selon la revendication 4, d'une surpression dans un tuyau (1) où circule un liquide électriquement conducteur, caractérisé en ce que les moyens de détection (11) du déplacement de l'organe d'obturation (9, 9′) comprennent une chambre (23; 23′) dans laquelle sont disposées au moins deux électrodes (25, 25′; 27, 27′) espacées l'une de l'autre et en ce que l'extrémité aval du tube (7) débouche dans cette chambre (23), les électrodes (25, 25′; 27, 27′) étant reliées à un circuit électrique capable de générer ledit signal.

8. Dispositif de détection selon la revendication 7, caractérisé en ce que la première électrode (25) a la forme d'un manchon définissant au moins une partie des parois de ladite chambre (23) et en ce que la seconde électrode (27) est logée à l'intérieur de la première et présente une cavité (41) débouchant dans la chambre (23), cette cavité (41) étant définie par une paroi annulaire (43),

9. Dispositif de détection selon la revendication 7, caractérisé en ce que chaque électrode (25′; 27′) a la forme d'une portion de cylindre coupée dans le sens de la longueur et disposée dans la paroi de la chambre (23′).

10. Dispositif de détection selon les revendications 7, 8 ou 9, caractérisé en ce que la chambre (23; 23′) présente un évent (57).

11. Dispositif de détection selon l'une quelconque des revendications 1 à 6, caractérisé en ce que les moyens de détection (11) du déplacement de l'organe d'obturation (9, 9′) comprennent un capteur photoélectrique (64) disposé sur le trajet dudit organe d'obturation (9; 9′) pour être activé au passage de celui-ci.

12. Dispositif de détection selon l'une quelconque des revendications 1 à 11, caractérisé en ce que l'organe d'obturation (9, 9′) est réalisé dans un matériau de type médical, stérilisable et chimiquement neutre vis-à-vis du fluide (3).

13. Dispositif de détection selon l'une quelconque des revendications 1 à 12, caractérisé en ce que l'organe d'obturation (9) est réalisé en métal.

14. Dispositif de détection selon l'une quelconque des revendications 1 à 12, caractérisé en ce que l'organe d'obturation (9′) est réalisé en une matière plastique telle que le polytétrafluoroéthylène, en matière céramique ou en verre.

15. Dispositif de détection selon la revendication 1, 2, 3, 4, 5, 6 12 ou 13, caractérisé en ce que les moyens de détection (11) du déplacement l'organe d'obturation (9) comprennent un capteur magnétique ou électromagnétique (69, 75) disposé sur le trajet dudit organe d'obturation pour être activé au passage de celui-ci.

16. Dispositif de détection selon la revendication 15, caractérisé en ce que deux organes d'obturation (9; 9′) sont placés dans le tube (7), le premier organe (9′) réalisé en matériau de type médical étant placé dans la partie amont dudit tube (7) et le second organe (9) réalisé en métal étant placé dans la partie aval du tube (7).

17. Dispositif de détection selon l'une quelconque des revendications 1 à 16, caractérisé en ce que le signal généré par les moyens de détection (11) du déplacement de l'organe d'obturation (9; 9′) est capable de déclencher une alarme (116).

18. Dispositif de détection selon l'une quelconque des revendications 1 à 17, caractérisé en ce qu'il comprend des moyens de maintien (49) de l'organe d'obturation (9, 9′) dans sa position de fonctionnement normal (A), ces moyens (49) étant effaçables et permettant d'autoriser la mise en marche dudit dispositif.

19. Dispositif de détection selon la revendication 18, caractérisé en ce que les moyens de maintien (49) comprennent un piston (51) engagé dans le tube (7) coaxialement à celui-ci.

20. Pompe entraînée par un moteur (109) comportant un dispositif de détection suivant l'une quelconque des revendications précédentes, ce dispositif de détection étant associé au tuyau d'alimentation (105) de cette pompe, caractérisé en ce que le signal généré par les moyens de détection (11) du déplacement de l'organe d'obturation (9; 9′) est capable de commander l'arrêt immédiat dudit moteur (109).

21. Pompe suivant la revendication 20, caractérisé en ce qu'elle est de type médicale et en ce que ledit fluide (3) est un médicament.

## Patentansprüche

1. Vorrichtung zum Erkennen eines Überdrucks in einer Leitung (1), in der ein Fluid (3) zirkulieren kann, welche Vorrichtung dazu bestimmt ist, ein Signal zu erzeugen, sobald eine Überdruckschwelle überschritten wird, umfassend:
- Mittel (5) zum Abzweigen einer Partie des Fluids (3), das sich in der Leitung (1) befindet,
- mindestens ein Rohr (7), in welchem sich mindestens ein Absperrorgan (9; 9′) befindet, welches Rohr mit seinem stromaufliegenden Ende mit den Abzweigmitteln (5) verbunden ist, wobei eines der das Rohr (7) und das Absperrorgan (9; 9′) bildenden Elemente elastisch deformierbar ist zum Halten des Absperrorgans in dem Rohr in einer Normalfunktionsposition (A) dank den Reibungskräften, die an den Kontaktoberflächen (21) erzeugt werden, während das Absperrorgan (9; 9′) die Normalfunktionsposition (A) verläßt, sobald in der Leitung (1) ein Überdruck vorliegt, der in der Lage ist, die genannten Reibungskräfte zu überwinden, und
- Erkennungsmittel (11) für die Verlagerung des Absperrorgans (9; 9′), welche das Signal erzeugen, sobald sie die Verlagerung des Absperrorgans (9; 9′) erkennen.

2. Erkennungsvorrichtung nach Anspruch 1, dadurch gekennzeichnet, daß das Rohr (7) aus einem elastischen Material hergestellt ist und daß das Absperrorgan (9; 9′) einen nicht deformierbaren Wälzkörper umfaßt, bemessen für die Reibung in dem Rohr (7).

3. Erkennungsvorrichtung nach Anspruch 2, dadurch gekennzeichnet, daß der Wälzkörper (9; 9′) eine Kontaktfläche (21) mit dem Rohr (7) mit einem Durchmesser besitzt, der mindestens gleich dem Durchmesser dieses Rohres ist.

4. Erkennungsvorrichtung nach Anspruch 2, dadurch gekennzeichnet, daß der Durchmesser des Wälzkörpers (9; 9′) in Höhe der Kontaktfläche (21) mit dem Rohr (7) größer ist als der Innendurchmesser dieses letzteren, um das Fluid (3), das sich in der stromaufliegenden Partie des Rohres (7) befindet, am Eindringen in die stromabliegende Partie (19) dieses letzteren zu hindern.

5. Erkennungsvorrichtung nach einem der Ansprüche 1 bis 4, dadurch gekennzeichnet, daß das Absperrorgan (9; 9′) die Form einer Kugel aufweist.

6. Erkennungsvorrichtung nach einem der Ansprüche 1 bis 5, dadurch gekennzeichnet, daß die Abzweigmittel (5) ein T-Stück umfassen, dessen beide Zweige (13; 15) in die Leitung (1; 1AM, 1AV) einfügbar sind und dessen dritter Zweig (17) mit dem stromaufliegenden Ende des Rohres (7) verbunden ist.

7. Erkennungsvorrichtung nach Anspruch 4 eines Überdrucks in einer Leitung (1), in der eine elektrisch leitende Flüssigkeit strömt, dadurch gekennzeichnet, daß die Erkennungsmittel (11) der Verlagerung des Absperrorgans (9; 9′) eine Kammer (23; 23′) umfassen, in der mindestens zwei Elektroden (25, 25′; 27, 27′) im Abstand voneinander angeordnet sind, und daß das stromabliegende Ende des Rohres (7) in diese Kammer (23) mündet, wobei die Elektroden (25, 25′; 27, 27′) mit einem elektrischen Schaltkreis verbunden sind, der zum Erzeugen des genannten Signals in der Lage ist.

8. Erkennungsvorrichtung nach Anspruch 7, dadurch gekennzeichnet, daß die erste Elektrode (25) die Form einer Hülse aufweist, welche mindestens eine Partie der Wandungen der Kammer (23) begrenzt, und daß die zweite Elektrode (27) im Inneren der ersten positioniert ist und einen Hohlraum (41) aufweist, der in die Kammer (23) mündet, welcher Hohlraum (41) durch eine ringförmige Wandung (43) begrenzt wird.

9. Erkennungsvorrichtung nach Anspruch 7, dadurch gekennzeichnet, daß jede Elektrode (25′; 27′) die Form eines Teils eines in Längsrichtung aufgeschnittenen Zylinders besitzt und in der Wandung der Kammer (23′) angeordnet ist.

10. Erkennungsvorrichtung nach Ansprüchen 7, 8 oder 9, dadurch gekennzeichnet, daß die Kammer (23; 23′) eine Belüftung (57) aufweist.

11. Erkennungsvorrichtung nach einem der Ansprüche 1 bis 6, dadurch gekennzeichnet, daß die Erkennungsmittel (11) der Verlagerung des Absperrorgans (9; 9′) einen photoelektrischen Sensor (64) umfassen, der über der Bahn des Absperrorgans (9; 9′) angeordnet ist, um bei Durchtritt desselben aktiviert zu werden.

12. Erkennungsvorrichtung nach einem der Ansprüche 1 bis 11, dadurch gekennzeichnet, daß das Absperrorgan (9; 9′) aus einem für medizinische Zwecke geeigneten, sterilisierbaren und chemischen, gegenüber dem Fluid (3) neutralen Material hergestellt ist.

13. Erkennungsvorrichtung nach einem der Ansprüche 1 bis 12, dadurch gekennzeichnet, daß das Absperrorgan (9) aus Metall hergestellt ist.

14. Erkennungsvorrichtung nach einem der Ansprüche 1 bis 12, dadurch gekennzeichnet, daß das Absperrorgan (9′) aus einem Kunststoffmaterial, wie Polytetrafluorethylen, aus keramischem Material oder aus Glas hergestellt ist.

15. Erkennungsvorrichtung nach Anspruch 1, 2, 3, 4, 5, 6, 12 oder 13, dadurch gekennzeichnet, daß die Erkennungsmittel (11) für die Verlagerung des Absperrorgans (9) einen magnetischen oder elektromagnetischen Sensor (69, 75) umfassen, angeordnet über der Bahn des Absperrorgans, um bei dessen Durchtritt aktiviert zu werden.

16. Erkennungsvorrichtung nach Anspruch 15, dadurch gekennzeichnet, daß zwei Absperrorgane (9; 9′) in dem Rohr (7) angeordnet sind, wobei das erste Organ (9′) aus einem für medizinische Zwecke geeigneten Material hergestellt ist und in der stromaufliegenden Partie des Rohres (7) angeordnet ist und das zweite Organ (9) aus Metall hergestellt ist und in der stromabliegenden Partie des Rohres (7) angeordnet ist.

17. Erkennungsvorrichtung nach einem der Ansprüche 1 bis 16, dadurch gekennzeichnet, daß das von den Erkennungsmitteln (11) der Verlagerung des Absperrorgans (9; 9′) gelieferte Signal für das Auslösen eines Alarms (116) geeignet ist.

18. Erkennungsvorrichtung nach einem der Ansprüche 1 bis 17, dadurch gekennzeichnet, daß sie Haltemittel (49) für das Absperrorgan (9; 9′) in seiner Normalfunktionsposition (A) umfassen, welche Mittel (49) unterdrückbar sind und es ermöglichen, die Inbetriebnahme der Vorrichtung zu autorisieren.

19. Erkennungsvorrichtung nach Anspruch 18, dadurch gekennzeichnet, daß die Haltemittel (49) einen Kolben (51) umfassen, der in das Rohr (7) koaxial zu diesem eingreift.

20. Von einem Motor (109) angetriebene Pumpe mit einer Erkennungsvorrichtung gemäß einem der vorangehenden Ansprüche, wobei diese Erkennungsvorrichtung der Speiseleitung (105) dieser Pumpe zugeordnet ist, dadurch gekennzeichnet, daß das von den Erkennungsmitteln (11) der Verlagerung des Absperrorgans (9, 9′) gelieferte Signal in der Lage ist, das sofortige Anhalten des Motors (109) zu steuern.

21. Pumpe nach Anspruch 20, dadurch gekennzeichnet, daß sie vom medizinischen Typ ist und daß das Fluid (3) ein Medikament ist.

## Claims

1. Detector for over-pressure in a pipe (1) in which a fluid (3) may flow, this detector being adapted to produce a signal when an over-pressure threshold is reached and comprising :
- means (5) for deriving a fraction of said fluid (3) from the pipe (1),
- at least one tube (7) wherein there is fitted at least one obturating member (9; 9′), such tube being connected at its upstream end to said deriving means (5), one of the elements forming said tube (7) and said obturating member (9; 9′) being resiliently deformable to retain said obturating member in said tube in a normal operating position (A) by means of frictional forces generated at their contacting surfaces (21), the obturating member (9; 9′) leaving the normal operating position (A) when said pipe (1) is subjected to an over-pressure sufficient to overcome the frictional forces, and
- means (11) for detecting the displacement of said obturating member (9; 9′) and for generating said signal in response to detecting displacement of the obturating member (9, 9′).

2. Detector according to claim 1, characterized in that said tube (7) is made of an elastic material and in that said obturating member (9, 9′) is a non-deformable body of revolution friction fitted in said tube (7).

3. Detector according to claim 2, characterized in that said body of revolution (9; 9′) has at the contact surface (21) with said tube (7) a diameter at least equal to the diameter of said tube.

4. Detector according to claim 2, characterized in that the diameter of the body of revolution (9, 9′) at the contact surface (21) with said tube (7) is greater than the inner diameter of the latter to prevent fluid (3) in the upstream part of the tube (7) from penetrating into the downstream part (19) of the latter.

5. Detector according to any one of the preceding claims, characterized in that the obturating member (9, 9′) is a ball.

6. Detector according to any one of the preceding claims, characterized in that the deriving means (5) comprise a T junction having two branches (13; 15) adapted to be connected in said pipe (1; 1AM, 1AV) and a third branch (17) connected to the upstream end of said tube (7).

7. Detector according to claim 4, for over-pressure in a pipe (1) in which an electrically conductive liquid flows, characterized in that the means (11) for detecting the displacement of the obturating member (9, 9′) comprise a chamber (23; 23′) in which at least two electrodes (25, 25′; 27, 27′) are spaced apart from one another and in that the downstream end of the tube (7) leads into this chamber (23), the electrodes (25, 25′; 27, 27′) being connected to an electric circuit able to generate said signal.

8. Detector according to claim 7, characterized in that the first electrode (25) is in the form of a sleeve defining at least a part of the walls of said chamber (23) and in that the second electrode (27) is housed inside the first and has a cavity (41) leading into the chamber (23), such cavity (41) being defined by an annular wall (43).

9. Detector according to claim 7, characterized in that each electrode (25′; 27′) has the shape of a segment of a cylinder cut lengthwise and arranged in the wall of the chamber (23′).

10. Detector according to claims 7, 8 or 9, characterized in that the chamber (23; 23′) has venting means (57).

11. Detector according to any one of claims 1 to 6, charac-terized in that the means (11) for detecting displacement of the obturating member (9, 9′) comprise a photoelectric sensor (64) arranged along the path of said obturating member (9; 9′) to be actuated by displacement thereof.

12. Detector according to any one of claims 1 to 11, characterized in that the obturating member (9, 9′) is made of a sterilizable medical material that is chemically neutral to the flowing fluid (3).

13. Detector according to any one of claims 1 to 12, characterized the obturating member (9) is made of metal.

14. Detector according to any one of claims 1 to 12, characterized in that the obturating member (9′) is made of plastic material such as polytetrafluoroethylene, ceramic material or glass.

15. Detector according to claim 1, 2, 3, 4, 5, 6, 12 or 13, characterized in that the means (11) for detecting displace-ment of the obturating member (9) comprises a magnetic or an electro-magnetic sensor (69, 75) arranged along the path of said obturating member to be actuated upon displacement of the latter.

16. Detector according to claim 15, characterized in that two obturating members (9; 9′) are placed in the tube (7), the first member (9′) made of medical material being arranged in the upstream part of said tube (7) and the second member (9) made of metal being arranged in the downstream part of the tube (7).

17. Detector according to any one of claims 1 to 16, characterized in that the signal generated by the means (11) for detecting displacement of the obturating member (9; 9′) is able to set off an alarm (116).

18. Detector according to any one of claims 1 to 17, characterized in that it comprises means (49) for maintaining the obturating member (9, 9′) in its position for normal operation (A), these means (49) being withdrawable to set said detector in its operative condition.

19. Detector according to claim 18, characterized in that the maintaining means (49) comprise a piston (51) arranged coaxially in the tube (7).

20. Pump driven by a motor (109) comprising a detector according to any one of the preceding claims, such detector being arranged in the supply pipe (105) of this pump, charac-terized in that the signal generated by the means (11) for detecting displacement of the obturating member (9; 9′) is able to control immediate stopping of said motor (109).

21. Pump according to claim 20, characterized in that it is a medical pump and in that said fluid (3) is a medication.
